# EUROPEAN PATENT APPLICATION

(11) **EP 2 229 883 A1**
(43) Date of publication of application: **22.09.2010**
(21) Application number: 10380036.3
(22) Date of filing: 16.03.2010
(51) Int. Cl.: A61B 5/103

(54) **Instrument for the objective measurement of the shoulder range of motion**

(30) Priority: 17.03.2009 ES 200900804
(71) Applicant: Fundación para el Progreso del Soft Computing, 33600 Mieres Asturias (ES)
(72) Inventor: Fernández Jose, Manuel, 28034 Madrid (ES); Trivino, Gracián, 33600 Mieres, Asturias (ES)

(57) **Abstract**

Summary (200 words max)

The device provides two types of measurement: an objective evaluation of the range of motion of the shoulder and an assessment of the limitation of the movement of the shoulder by compensatory movements with the spine or trunk. The instrument consists of two clinometers and a storage and visualisation device connected by means of a wireless connection. One of the clinometers is fixed on the patient's arm (wrist) and the other on the back between the shoulder blades. Each clinometer sends to the storage and visualisation device the angle between its longitudinal axis and the gravity vector.

For the objective evaluation of the range of motion of the shoulder, the patient lays on the examination couch (dorsal decubitus position) and the angles of mobility of the shoulder are studied. To assess the limitation of the motion of the shoulder by compensatory movements with the spine or trunk, the patient stands in erect position with the arms hanging at sides along the trunk. The patient must separate the extended arm from the trunk (making an internal rotational movement of the thumb oriented towards the floor) until it is parallel to the ground.

## Description

### State of the technique

A device to provide an objective measure of the degree of mobility of the glenohumeral joint and the impact of its malfunction in the spine and in the body general position is not currently available. This type of pathology is very frequent in working, sports and leisure activities.

There is a type of devices to measure the displacement of the glenohumeral joint when a force is applied. (See registered USA patents with codes nº 6390994 and nº 6551258). Nevertheless, these devices are aimed to measure the laxity and instability of this joint with respect to an externally applied force while the present invention is aimed to measure the shoulder mobility when it is moved by the patient without external forces.

### Invention explanation

The invention consists of three main components: The clinometer attached to the arm, the clinometer attached to the back and the device to store and visualise the data.

Each clinometer sends the measure of the angle between its longitudinal axis and the gravity vector to the storage and visualisation device. This information appears in the display to be observed by both the specialist and the patient.

The invention provides an objective measure of the standard shoulder mobility angles recommended by the society of American Shoulder and Elbow Surgeons (ASES):
a.- Anterior elevation of the arm of the patient in dorsal decubitus position.
b.- Active external rotation with the arm closed to the body.
c.- Active internal and external rotation with the arm in horizontal position (angle of 90° with the body).

The measurement and storage of these angles permits to compare the differences before and after any kind of treatment (medical, rehabilitation or surgical).

Additionally, the invention allows detecting the limits in the angular separation of the arms from the body and its consequences on the balance and position while standing upright.

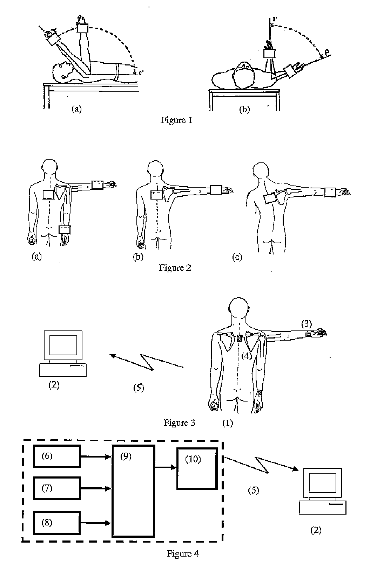

For the measurement of the angles' mobility, the patient is placed lying down (prone position) on the examination couch (Figure 1).

In order to make the assessment of the limitation of the shoulder motion by doing compensatory movements with the spine or trunk, the patient is placed standing with his arms resting along the trunk. The patient is asked to separate the extended arm from the body (at internal rotation in which he must place the thumb of the hand facing the floor) until it reaches the horizontal position (angle of 90°with the body). The arm movement away from the trunk in a normal shoulder is carried out initially at the expense of the glenohumeral joint (figure 2a) and is then supplemented by a scapular-thoracic movement (or displacement of the scapula on the thorax wall, see figure 2b). When the shoulder joint has some pathology, the motion of the glenohumeral joint is limited, the separation is then done at the expense of the scapular-thoracic joint. In this case, this is followed by a tilt of the spine (figure 2c) to help achieve the highest elevation of the arm in an attempt to compensate the movement that should take place at the glenohumeral joint.

### Clinometer attached to the arm

This is a box no larger than 150 x 100 x 15 mm which is fixed at the end of the arm near the wrist using a glove or a Velcro tape.

The box contains an electronic circuit that includes three orthogonal accelerometers, a microcontroller, a wireless information transmission system and a battery to power the circuit.

The Clinometer attached to the Arm performs the following functions:
- To measure the angle between the longitudinal axis of the sensor (forearm direction) and the gravity force.
- To send this information to the storage and visualisation system via wireless connection.

### Clinometer attached to the back

This device is identical to the clinometer attached to the arm (in size, structure and components) and its function is similar. In this case, the clinometer should be fixed between the shoulder blades (at the confluence of the lines traced out by the spines of the shoulder blades, see figure 3) with a harness or some sticking plaster.

The clinometer attached to the back performs the following functions:
- To measure the angle formed by the axis of the spine with the gravity force vector and to determine the angle of separation of the arm when the spine begins to move.
- To send this information to the storage and visualisation system via wireless connection in order to assess whether a movement of the spine to raise the arm is used or not.

### Storage and visualisation Device

The device consists of a wireless receiver, a memory and a display and it can be made with a personal computer (PDA or similar) provided with a wireless connection and controlled by a computer program. This device stores the data received from the clinometers and shows it on the display. The display can show a curve with the evolution of the measured angles and a graph with the position of the patient.

### Description of the drawings

Figure 1 shows two examples of measurement of the angles of mobility of the shoulder with the patient lying down (in prone position) on the examination couch.
Figure 2 shows a sequence with the movement of the arm in a blocked shoulder causing the inclination of the spine.
Figure 3 shows a scheme of the complete system providing its principal components. Figure 4 shows the elements integrated in a clinometer. In these figures the following elements are identified:
   (1) Pacient
   (2) Display
   (3) Clinometer attached to the arm
   (4) Clinometer attached to the spine
   (5) Wireless connection
   (6) (7) (8) Orthogonal acceleration sensors
   (9) Microprocessor
   (10) Wireless transmission module

### Detailed presentation of at least one Implementation of the invention

One possible implementation of the invention is based on the use of the following materials:
Clinometers: The electronic circuit is built around a PIC18F2525 microcontroller from the company Microchip. Motion sensors are three ADXL202 accelerometers from Analog Devices placed in three perpendicular axes. For the wireless communication a EYMF2CAMM-XX Bluetooth module of BT Designer Company is being used.

The storage and display system can be made using a personal computer. The operating system may be Windows from Microsoft and the program for creating the storage and visualisation device can be programmed with Visual C++ tool also from Microsoft. The Bluetooth connection can be achieved with a commercial USB-Bluetooth converter.

### Industrial application

This invention can be used at the clinic of medical specialists (orthopaedists), general doctors, sports physicians or rehabilitation experts. The applications are early diagnosis and objective assessment in the monitoring of the evolution in the treatment of shoulder pathologies that limit the glono-humeral mobility. The invention is a fast and safe system requiring no special installation or preparation on the part of the patient. As it is free of ionizing radiation for the patient, the medical examination may be repeated as many times as needed.

## Claims

1. Instrument for measuring the mobility of the shoulder which comprises: two clinometers with three acceleration sensors (6) (7) (8) placed in orthogonal vectors, a wireless communication module (10), an electric energy accumulator, an electronic circuit based on a microprocessor (9) able to measure the tilt sensor and send the measurement, these components are enclosed in a box adjustable to the arm or back of the patient; a storage and visualisation system with a reception module for wireless communications, a display to view the measures, a data storage system, a computer program that displays and stores the measurements, a user interface (keyboard, mouse, touch screen or similar) to start and close the program. The instrument provides measures that allow the user to know at the same time both the relative tilt angles of the back and arm.

2. The instrument of claim 1 which comprises a single adjustable arm clinometer to measure the patient's arm mobility by fixing the position of the patient's back (e.g. lying on an exploration examination couch).
